(19) Europäisches Patentamt
European Patent Office
Office européen des brevets

(11) Publication number: **0 164 765**
**A1**

(12) # EUROPEAN PATENT APPLICATION

(21) Application number: 85200531.3

(22) Date of filing: 19.02.82

(51) Int. Cl.[4]: C 07 D 513/04
A 61 K 31/425, A 61 K 31/415

(30) Priority: 19.02.81 JP 23515/81
21.04.81 JP 59990/81
02.10.81 JP 157010/81

(43) Date of publication of application:
18.12.85 Bulletin 85/51

(84) Designated Contracting States:
AT BE CH DE FR GB IT LI LU NL SE

(60) Publication number of the earlier application in accordance with Art. 76 EPC: 0 059 090

(71) Applicant: YAMANOUCHI PHARMACEUTICAL CO. LTD.
No. 5-1 Nihonbashi-Honcho, 2-chome Chuo-ku
Tokyo(JP)

(72) Inventor: Kubo, Kazuo
No. 21-4, Negishi 2-chome
Urawa-shi Saitama(JP)

(72) Inventor: Isomura, Yasuo
No. 12-11-919, Nishioku 4-chome
Arakawa-ku Tokyo(JP)

(72) Inventor: Sakamoto, Shuichi
No. 2-1258-201, Ogawacho
Kodaira-shi Tokyo(JP)

(72) Inventor: Homma, Hiroshige
No. 139-2, Kamikomachi
Omiya-shi Saitama(JP)

(74) Representative: Geering, Keith Edwin et al,
REDDIE & GROSE 16 Theobalds Road
London WC1X 8PL(GB)

(54) **3,5-Di-Tert-Butyl-4-hydroxyphenyl-substituted heterocyclic compounds.**

(57) Anti-rheumatic pharmaceutical compounds of formula

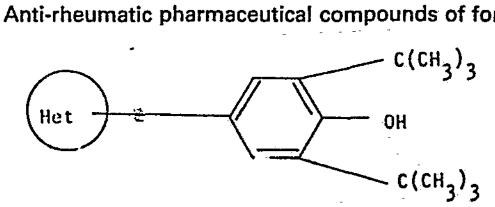

and salts thereof, where

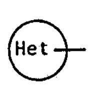

is a substituted or un-substituted (2,3-dihydro)imidazo [2,1-b] thiazole ring.

Croydon Printing Company Ltd.

## 3,5-DI-TERT-BUTYL-4-HYDROXYPHENYL-SUBSTITUTED HETEROCYCLIC COMPOUNDS

This invention relates to 3,5-di-tert-butyl-4-hydroxyphenyl-substituted heterocyclic compounds, their preparation, and their pharmaceutical use.

According to the invention there are provided the 3,5-di-tert-butyl-4-hydroxyphenyl-substituted heterocyclic compounds of formula I, and their salts :

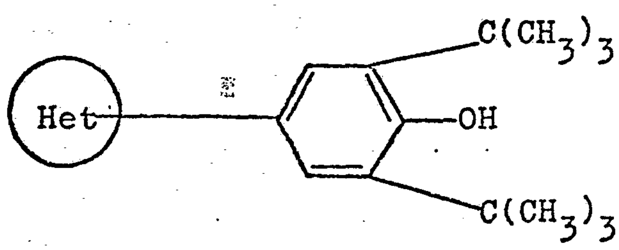

I

wherein Het- is a heterocyclic group of the formula

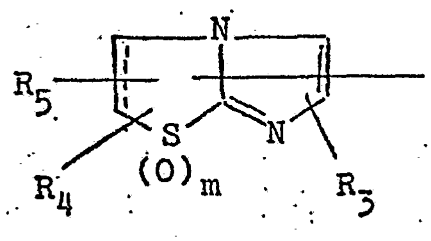

wherein

$R_3$, $R_4$, and $R_5$ are the same or different and selected from hydrogen, halogen, $C_1$ to $C_7$ alkyl groups, hydroxy $C_1$ to $C_7$ alkyl groups, amino $C_1$ to $C_7$ alkyl groups, aryl groups, $C_1$ to $C_7$ alkoxy-substituted aryl groups, $C_1$ to $C_7$ alkanoyl groups, $C_1$ to $C_7$ alkylthio groups, $C_1$ to $C_7$ alkoxy groups, aryl-substituted $C_1$ to $C_7$ alkyl groups, and cyano and thiocyanato groups; m is 0, 1, or 2; and the dotted line means the presence or absence of a double bond.

In the following description, the term "lower" means a straight or branched carbon chain of 1-7 carbon atoms. For example, "lower alkyl" includes methyl, ethyl, propyl, isopropyl, butyl, isobutyl, tert-butyl, heptyl, and hexyl; "lower aralkyl" which means aryl-substituted lower alkyl, includes benzyl and phenethyl; "lower alkoxy" includes methoxy, ethoxy, propoxy, isopropoxy, butoxy, and hexyloxy; "lower alkylthio" includes methylthio, ethylthio, propylthio and isopropylthio; and "lower alkanoyl" includes formyl, acetyl, propionyl, isopropionyl, butyryl and hexanoyl. The term "amino" in "amino $C_1$-$C_7$ alkyl" means not only $-NH_2$ but also mono- and di-alkylamino such as the methylamino, dimethylamino and diethylamino, and cyclic amino such as morpholino, pyrrolidino, piperidino, piperazino and 4-lower alkylpiperazino group. Furthermore, "aryl" includes phenyl and naphthyl etc, and "halogen" includes chlorine, bromine, iodine and fluorine.

The salts of this invention include the pharmaceutically acceptable acid addition salts, for example inorganic acids such as hydrochloric, hydrobromic, sulfuric, nitric and phosphoric acids.

Compounds of this invention have variously shown anti-inflammatory,anti-pyretic, analgesic, antiarthritic and immuno-regulatory activities.

Compounds of this invention show therapeutic and prophylactic effects on adjuvant-induced arthritis which is considered to be an animal model of human rheumatism, have analgesic and anti-inflammatory activities and, as a biochemical activity, inhibit prostaglandin formation; they are thus considered to be useful for the therapy and prophylaxis of human rheumatic disease. Moreover, compounds of this invention suppress Coomb's type III (Arthus reaction) and type IV (delayed type hypersensitivity) allergic reactions, as well as having a lipoxigenase suppressing activity, and acting as radical scavengers, unlike conventional (acidic) nonsteroidal anti-inflammatory antirheumatics as represented by indomethacin and diclofenac. The compounds of this invention thus seem to act as antirheumatics by a new mechanism.

Aryl substituted heterocyclic compounds having some anti inflammatory and/or anti rheumatic activity are known from the prior art e.g. Burgers Medicinal Chemistry, 4th Edn, Part III, Wolff, John Wiley & Sons 1981, pp 1251-1252 discusses Levamisole; EP-0013560 and EP-0013561 discuss methoxyphenyl-substituted tetrahydro imidazo thiazols; US 3578671 and US 4072689 discuss substituted oxazoles.

Phenyl substituted imidazo thiazoles have been generally described in EP-A2-0 000353 wherein the phenyl group may have substituents such as methyl, methoxy or halogen. There is no suggestion of mixed substitution, nor of the particular 3,5-di-tert-butyl-4-hydroxylphenyl group of the compounds of the present invention.

One feature of the compounds of this invention is that they are nonacidic nonsteroidal anti-inflammatory agents, as compared to the conventional, acidic non-steroidal anti-inflammatory agents such as indomethacin and diclofenac. Another feature is that the heterocyclic

ring is directly substituted by a 3,5-di-tert-butyl-4-hydroxyphenyl group. A third feature is that the basic substituted heterocyclic ring itself is selected from specific (2,3-dihydro)imidazo[2,1-b]thiazole rings as defined above.

As heterocyclic compounds the heterocyclic ring of which is directly substituted by a 3,5-di-tert-butyl-4-hydroxyphenyl group, there are known, for example, 2-(3,5-di-tert-butyl-4-hydroxyphenyl)benzoxazole compounds and 2-(3,5-di-tert-butyl-4-hydroxyphenyl)-benzothiazole compounds (West German Offenlegungsschrift 2,008,414); 2-(3,5-di-tert-butyl-4-hydroxyphenyl)-1,3-diphenylimidazolidine compounds (Belgian Patent No. 807,14); and 2-[2-(3,5-di-tert-butyl-4-hydroxyphenyl)-5-ethoxy-4-thiazolyl]acetic acid (Japanese Pat. Appln. Laid Open No. 7669/'78). The benzoxazole compounds and the benzothiazole compounds are used as antioxidants and the imidazolidine compounds are used as intermediates for plant protecting agents and dyes. The latter acidic acid compound is suggested for use as an anti-thrombotic agent, a hypolipaemic agent, and an anti-inflammatory agent, but there is no disclosure about its practical pharmacological effect as an anti-inflammatory agent and it is in any case an acidic non-steroidal anti-inflammatory agent and hence chemically different from the non-acidic compounds of this invention.

Throughout the structural formulae in this

specification, the same symbols retain the same meanings unless otherwise specified.

The compounds of this invention are of formula Ib:

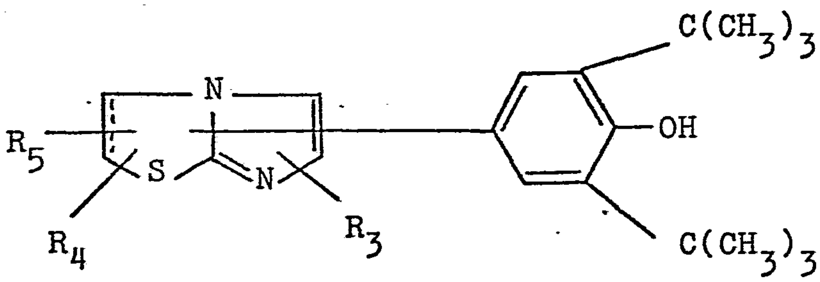

Ib

Particularly preferred compounds of formula Ib are as follows :

6-(3,5-di-tert-butyl-4-hydroxyphenyl)-5-methyl-2,3-dihydroimidazo[2,1-b] thiazole,

6-(3,5-di-tert-butyl-4-hydroxyphenyl)-2,3-dihydroimidazo[2,1-b] thiazole 1-oxide,

6-(3,5-di-tert-butyl-4-hydroxyphenyl)-2,3-dihydroimidazo[2,1-b] thiazole 1,1-dioxide, and

6-(3,5-di-tert-butyl-4-hydroxyphenyl)-2,3-dihydroimidazo[2,1-b] thiazole.

The compounds of formula Ib can be shown by formula Ib-1

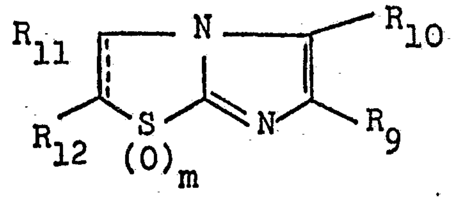

Ib-1

wherein one of $R_9$, $R_{10}$, $R_{11}$, and $R_{12}$ is the 3,5-di-tert-butyl-4-hydroxyphenyl group, and the others have the same significance as $R_3$, $R_4$, and $R_5$ above; these compounds can be prepared by the following methods:

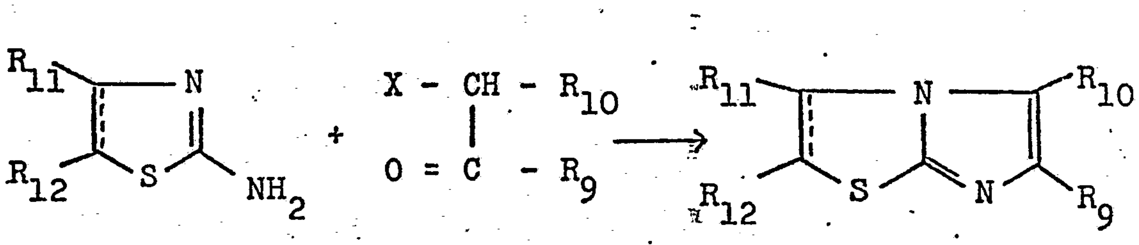

IIb IIIb Ib-2

wherein X is halogen.

Thus a compound Ib-2 can be prepared by heating a 2-aminothiazoline derivative or 2-aminothiazole derivative IIb with the corresponding amount of α-halo-carbonyl compound IIIb alone or in a suitable solvent such as listed under Ai) above. The reaction temperature and time are selected according to the solvent and starting materials used, but the reaction is advantageously performed under reflux.

0164765

Bii). Second production method (X is halogen).

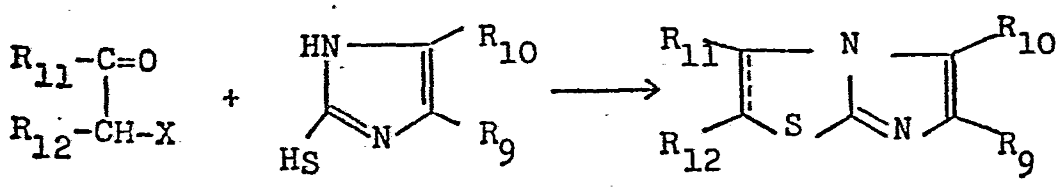

IVb Vb Ib-2

Thus a compound Ib-2 can also be prepared by reacting an α-halocarbonyl compound IVb and a corresponding amount of 2-mercaptoimidazole derivative Vb or a salt (e.g. an alkali metal salt) thereof alone or in a suitable solvent such as listed under Ai) above. The reaction usually proceeds at room temperature but may be performed under heating. The reaction time varies according to the solvent and starting materials used. When the compound Vb is supplied in the free form, it is preferably reacted with an alkali metal alcoholate to form a corresponding alkali metal salt for reaction with the compound of formula IVb.

This reaction is considered to proceed via an intermediate of formula VIb

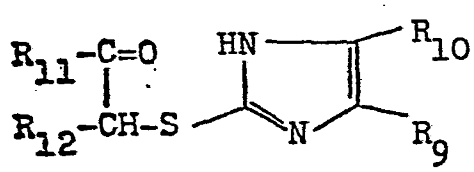

VIb

and, if necessary, the intermediate can be isolated. The intermediate VIb can be converted into the compound Ib-2 by treatment with an acid such as hydrochloric or acetic acid, or with phosphorus oxychloride.

A dihalogenoethane derivative IVb-1

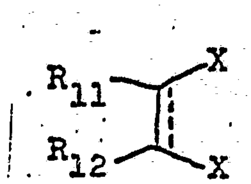

$IV_{b-1}$

can be used in method Bii) in place of the α-halocarbonyl starting compound IVb.

Biii) Other production methods:

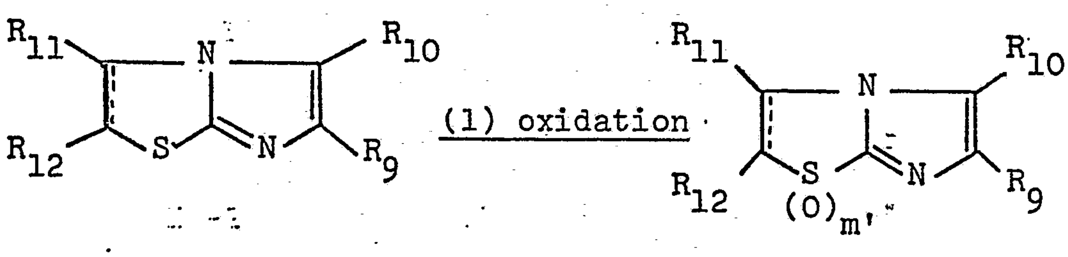


Ib-2 Ib-3

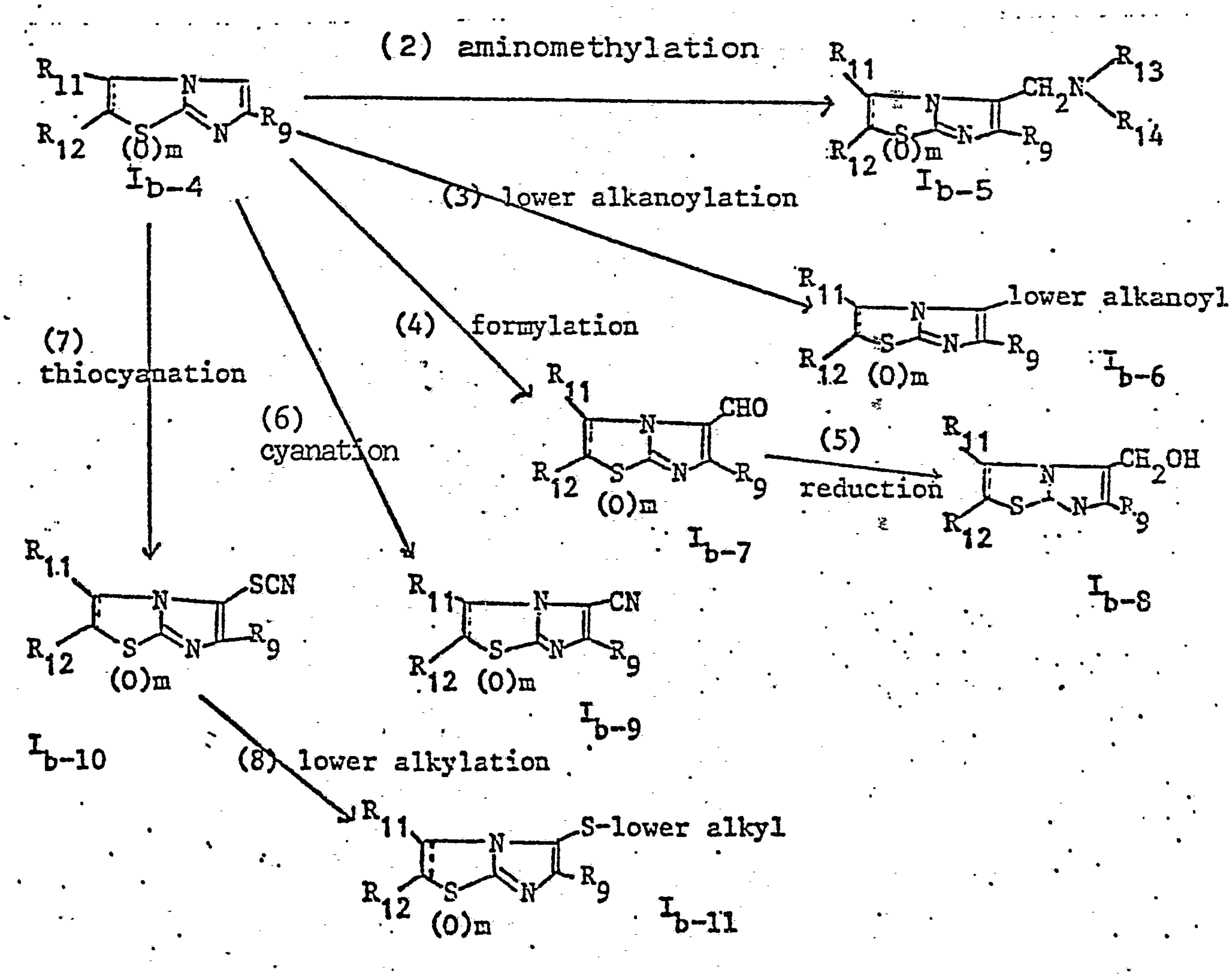


$I_{b-4}$ $I_{b-5}$ $I_{b-6}$ $I_{b-7}$ $I_{b-8}$ $I_{b-9}$ $I_{b-10}$ $I_{b-11}$

wherein m' is 1 or 2 and $R_{13}$ and $R_{14}$ are selected independently from hydrogen and lower alkyl groups or together form a cyclic amino group.

In oxidation method (1) an S-oxide compound Ib-3 can be prepared by reacting the corresponding thio compound Ib-2 with an oxidizing agent in a solvent such as chloroform, 1,2-dimethoxyethane or acetic acid in conventional manner. Suitable oxidizing agents include 10-40% hydrogen peroxide, perbenzoic acid and m-chloroperbenzoic acid. By suitably selecting the reaction conditions such as reaction time and temperature and the amount of the oxidizing agent, the monoxide (m' = 1) or dioxide (m' = 2) compound can be obtained as desired.

In aminomethylation method (2) an aminomethyl compound formula Ib-5 is formed by reacting a compound of formula Ib-4 with formaldehyde and an amine 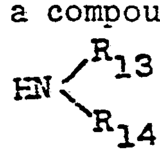 in a Mannich reaction.

In the alkanoylation method (3) a lower alkanoyl compound Ib-6 can be prepared by reacting a compound Ib-4 with a lower alkanoyl halide such as acetyl chloride and an acid anhydride such as acetic anhydride in conventional manner.

In method (4) a formyl compound Ib-7 can be prepared by reacting a compound Ib-4 with a complex (Vilsmeier reagent) of dimethylformamide (DMF) and phosphorus oxychloride or oxalyl chloride in conventional manner and then hydrolyzing the reaction product.

In reduction method (5) a hydroxymethyl compound

Ib-8 can be produced by reducing a formyl compound Ib-7 with a reducing agent such as sodium borohydride.

In cyanation method (6) a nitrile compound Ib-9 can be produced by reacting a compound Ib-4 with a Vilsmeier reagent as in method (4) and then reacting the reaction product with hydroxylamine

In thiocyanation method (7) a thiocyanate compound Ib-10 can be produced by reacting a compound Ib-4 with a thiocyanate such as sodium thiocyanate and bromine.

In lower alkylation method (8) a lower alkylthio compound Ib-11 can be produced by reacting a thiocyanate compound Ib-10 with a lower alkylating agent in conventional manner. Suitable alkylating agents include lower alkyl halides such as methyl iodide and ethyl iodide.

The compounds of this invention can be isolated and purified by conventional chemical operations such as concentration, recrystallization, and column chromatography.

The results of tests showing the pharmacological effects of compounds of this invention are as follows.

Effect on adjuvant-induced arthritis in rats:

Method: Male Sprague Dawley rats aged 7 weeks were used. Drugs were evaluated by two methodsas follows. All test drugs were suspended in water with 0.5% methylcellulose (0.5% MC) and administered orally (P.O)once a day.

(I) Therapeutic effect of drugs: arthritis was induced by a single injection of 0.1 ml of sterile

suspension of Mycobacterium butyricum (6mg/ml) in liquid paraffin into the tail skin of rats (day 0). After about 2 weeks, arthritic rats were selected and allocated into groups. Drugs were given daily thereafter for about 10 days. Foot thickness was measured with a dial thickness gauge both on the day of initial dosing and on the day after final dosing, and the change of foot thickness ($\Delta FT\ 10^{-2}mm$) was calculated as the difference between these two values. The results are shown in Table I. (N = number of animals). (a) Prophylactic effect of drugs: arthritis was induced by single subplanter injections of 0.05 ml of the suspension into the left hind paws of rats. Drugs were given daily for 21 days starting from the day (day 0) of injection of the suspension. The thicknesses of both rear feet, injected foot (left foor, $FT_L$) and uninjected foot (right foot, $FT_R$) were measured with a dial thickness gauge on days 0 and 21. The percent inhibition (I%) was calculated from the difference in increased foot thickness between the control and drug-treated groups. The results are shown in Table II. (N = number of animals).

Table I (Therapeutic effect)

| Drug | Dose (mg/kg/day P.O.) | N | Day16 - Day28 ⊿FT ($10^{-2}$mm) |
|---|---|---|---|
| 0.5% MC | - | 3 | 210 ± 91 |
| Indomethacin | 2 | 3 | -177 ± 41** |
| 6-(3,5-di-tert-butyl-4-hydroxyphenyl)-5-methyl-2,3-dihydroimidazo[2,1-b]-thiazole | 25 | 3 | -16 ± 27* |
| 6-(3,5-di-tert-butyl-4-hydroxyphenyl)-2,3-dihydroimidazo[2,1-b]thiazole 1-oxide | 25 | 3 | -245 ± 57** |
| 6-(3,5-di-tert-butyl-4-hydroxyphenyl)-2,3-dihydroimidazo[2,1-b]thiazole 1,1-dioxide | 25 | 3 | -141 ± 60** |

| Drug | Dose (mg/kg/day P.O.) | N | Day 17 - Day27 ⊿FT ($10^{-2}$mm) |
|---|---|---|---|
| 0.5% MC | - | 3 | 181 ± 7 |
| Indomethacin | 2 | 3 | -225 ± 61** |
| Diclofenac HCl | 2 | 3 | -83 ± 49* |
| 6-(3,5-di-tert-butyl-4-hydroxyphenyl)-2,3-dihydroimidazo[2,1-b]thiazole | 25 | 3 | -177 ± 36*** |

Significant difference from control (t-test)

* $P < 0.05$ ** $P < 0.01$ *** $P < 0.001$

0164765

| Drug | Dose (mg/kg/day P.O.) | N | Day 16 - Day 28 ΔFT (x $10^{-2}$ mm) |
|---|---|---|---|
| 0.5% MC | - | 16 | 171 ± 39 |
| Phenylbutazone | 50 | 9 | -133 ± 42*** |
| Indomethacin | 2 | 6 | -175 ± 44*** |
| 5,6-bis(p-methoxyphenyl)-2,3-dihydroimidazo[2,1-b]-thiazole(EP-A2-0 000 353, Ex. 1) | 25 | 3 | 18 ± 40* |

| Drug | Dose (mg/kg/day P.O.) | N | Day 14 - day 28 ΔFT (x $10^{-2}$ mm) |
|---|---|---|---|
| 0.5% MC | - | 6 | 293 ± 29 |
| Ibuprofen | 10 | 6 | 172 ± 20** |
| | 30 | 6 | 156 ± 22** |
| | 100 | 6 | - 53 ± 24*** |
| 4-(3,5-di-tert-butyl-4-hydroxyphenyl)-2-oxo-thiazoline | 30 | 3 | -150 ± 36*** |
| 4-(3,5-di-tert-butyl-4-hydroxyphenyl)-5-methyl-2-oxo-4-thiazoline | 30 | 3 | - 48 ± 53*** |
| 4-(3,5-di-tert-butyl-4-hydroxyphenyl)-5-methyl-2-oxo-4-imidazoline | 10 | 6 | 91 ± 35** |
| | 30 | 6 | -184 ± 12*** |
| | 100 | 5 | -199 ± 20*** |

** $P<0.01$, *** $P<0.001$

The preparation of compounds according to the invention is illustrated by the following Examples:

Example 1

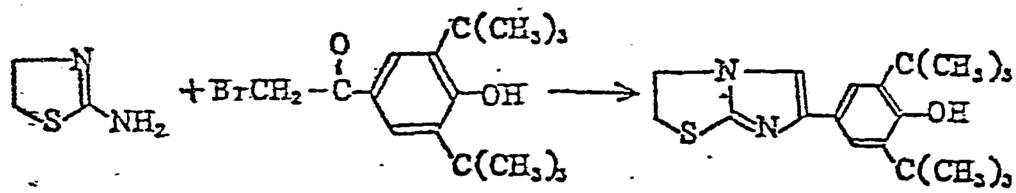

In 300 ml of methyl ethyl ketone was dissolved 10.2 g of 2-amino-2-thiazoline, and after 32.7 g of 3,5-di-tert-butyl-4-hydroxyphenacyl bromide was added portionwise to the solution followed by stirring for 30 minutes at room temperature, the resultant mixture was refluxed for one hour. Then, the solvent was distilled off under reduced pressure, the crystals thus formed were dissolved in 200 ml of ethanol, and the solution was refluxed for 4 hours. The reaction mixture was basified with the addition of aqueous ammonia and then 200 ml of water was added to the reaction mixture to precipitate crude crystals, which were recovered by filtration and recrystallized from ethanol to provide 23.5 g of 6-(3,5-di-tert-butyl-4-hydroxyphenyl)-2,3-dihydroimidazo[2,1-b]thiazole.

Melting point: 212-214°C.

0164765

Elemental analysis for $C_{19}H_{26}N_2OS$:

| | C(%) | H(%) | N(%) |
|---|---|---|---|
| Calculated: | 69.11 | 8.04 | 8.43 |
| Found: | 69.05 | 7.93 | 8.48 |

Example 2

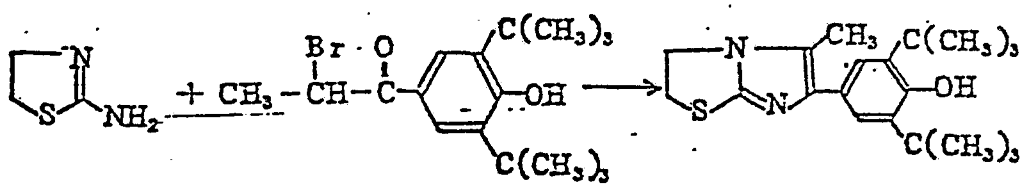

By following the same procedure as in Example 57, 6-(3,5-di-tert-butyl-4-hydroxyphenyl)-5-methyl-2,3-dihydroimiazo[2,1-b]-thiazole was produced.

Melting point: 258-261°C

Elemental analysis for $C_{20}H_{28}N_2OS$:

| | C(%) | H(%) | N(%) |
|---|---|---|---|
| Calculated: | 69.62 | 8.22 | 8.11 |
| Found: | 69.73 | 8.19 | 8.13 |

Example 3

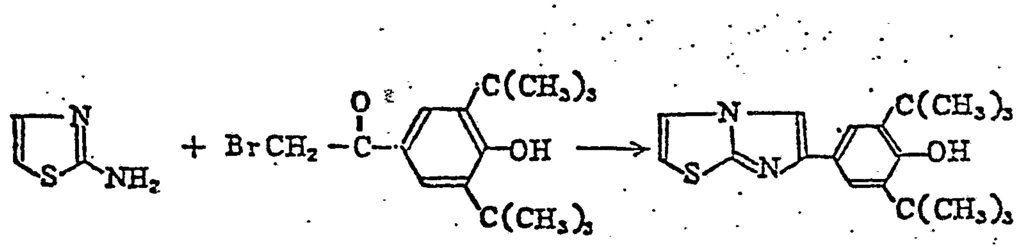

In 20 ml of ethanol were dissolved 1.2 g of 2-aminothiazole and 3.9 g of 3,5-di-tert-butyl-4-hydroxyphenacyl bromide, and the

solution was refluxed for 2 hours. The reaction mixture was neutralized by the addition of aqueous ammonia and added 30 ml of water. The crystals thus precipitated were recovered by filtration and recrystallized from ethanol to provide 1.5 g of 6-(3,5-di-tert-butyl-4-hydroxyphenyl)imidazo[2,1-b]-thiazole.

Melting point: 184-185°C

Elemental analysis for $C_{19}H_{24}N_2OS$:

| | C(%) | H(%) | N(%) |
|---|---|---|---|
| Calculated: | 69.31 | 7.65 | 8.38 |
| Found: | 69.48 | 7.36 | 8.53 |

Examples 4 - 8

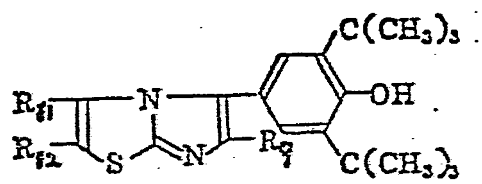

By following the same procedure as in Example 59, the following compounds were produced.

Example 4

Compound: The compound of the above formula wherein $R_7$ is $CH_3$, $R_{11}$ is $CH_3$, and $R_{12}$ is H.

Melting point (n-hexane-cyclohexane) : 188-190°C

Elemental analysis for $C_{21}H_{28}N_2OS$:

| | C(%) | H(%) | N(%) |
|---|---|---|---|
| Calculated: | 70.56 | 8.03 | 7.64 |
| Found: | 70.75 | 7.92 | 7.86 |

Example 5

Compound: The compound of the above formula wherein $R_9$ is $CH_3$, $R_{11}$ is $CH_3$, and $R_{12}$ is $CH_3$.

Melting point: 208-210°C (n-hexane)

Elemental analysis for $C_{22}H_{30}N_2OS.1/4H_2O$:

| | C(%) | H(%) | N(%) |
|---|---|---|---|
| Calculated: | 70.67 | 8.27 | 7.39 |
| Found: | 71.31 | 8.16 | 7.56 |

Example 6

Compound: The compound of the above formula wherein $R_9$ is H, $R_{11}$ is H, and $R_{12}$ is Br.

Melting point: 174-176°C (n-hexane-cyclohexane)

Elemental analysis for $C_{19}H_{23}BrN_2OS$:

| | C(%) | H(%) | N(%) |
|---|---|---|---|
| Calculated: | 56.40 | 5.92 | 6.55 |
| Found: | 56.02 | 5.69 | 6.88 |

Example 7

Compound: The compound of the above formula wherein $R_9$ is H, $R_{11}$ is H, and $R_{12}$ is $CH_3S$.

Melting point: 131-132°C (n-hexane-cyclohexane)

Elemental analysis for $C_{20}H_{26}N_2OS_2$:

| | C(%) | H(%) | N(%) |
|---|---|---|---|
| Calculated: | 63.95 | 7.09 | 7.62 |
| Found: | 64.14 | 7.00 | 7.48 |

Example 8

Compound: The compound of the above formula wherein $R_9$ is H, $R_{11}$ is a phenyl group, and $R_{12}$ is $CH_3$.

Melting point: 229-231°C (n-hexane)

Elemental analysis for $C_{26}H_{30}N_2OS$:

| | C(%) | H(%) | N(%) |
|---|---|---|---|
| Calculated: | 74.34 | 7.60 | 6.49 |
| Found: | 74.60 | 7.22 | 6.69 |

Example 9

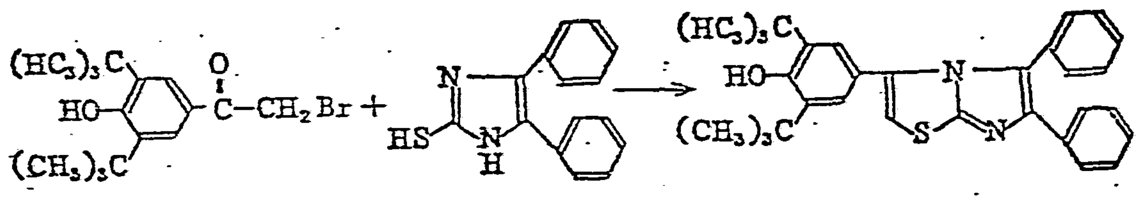

In 20 ml of ethanol was dissolved 0.11 g of metallic sodium and after adding 1 g of 4,5-diphenyl-2-mercaptoimidazole to the solution followed by stirring several minutes, 1 g of 3,5-di-tert-butylphenacyl bromide was added thereto followed by stirring for one hour at room temperature. The reaction mixture was concentrated under reduced pressure and then water was added to the residue. The crystals thus formed were recovered by filtration, dried, dissolved in 20 ml of phosphorus oxychloride, and the solution was refluxed for 14 hours. The reaction mixture was concentrated under reduced pressure and after adding water to the residue followed by neutralization with potassium carbonate, the product was extracted with chloroform. The extract was dried and concentrated. The residue was subjected to silica gel column chromatography and the product was eluted with chloroform. The crude crystals thus obtained were recrystallized from ethanol to

provide 0.8 g of 3-(3,5-di-tert-butyl-4-hydroxyphenyl)-5,6-diphenylimidazo[2,1-b]thiazole.

Melting point: 237-238°C

Elemental analysis for $C_{31}H_{32}N_2OS$:

| | C(%) | H(%) | N(%) | S(%) |
|---|---|---|---|---|
| Calculated: | 77.47 | 6.57 | 5.70 | 6.48 |
| Found: | 77.46 | 6.71 | 5.83 | 6.67 |

Example 10

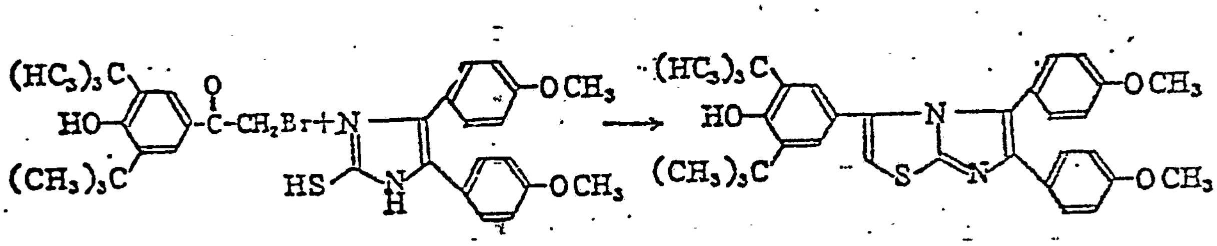

By following the same procedure as in Example 9, 3-(3,5-di-tert-butyl-4-hydroxyphenyl)-5,6-bis(p-methoxyphenyl)-imidazo[2,1-b]thiazole was produced.

Melting point: 236-237°C

Elemental analysis for $C_{33}H_{36}N_2SO_3$:

| | C(%) | H(%) | N(%) | S(%) |
|---|---|---|---|---|
| Calculated: | 72.92 | 6.53 | 5.32 | 6.42 |
| Found: | 73.30 | 6.71 | 5.18 | 5.93 |

Example 11

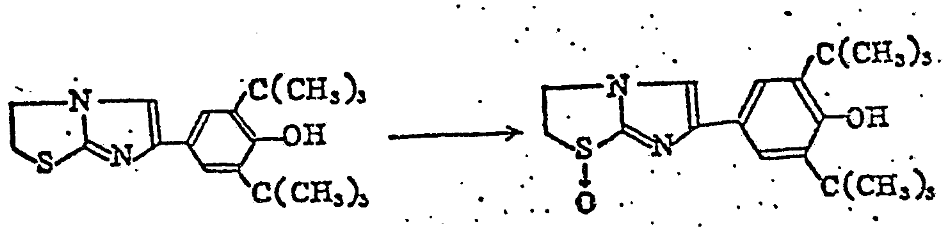

0164765

In 10 ml of chloroform was dissolved 1.6 g of 6-(3,5-di-tert-butyl-4-hydroxyphenyl)-2,3-dihydroimidazo[2,1-b]thiazole and after adding thereto 1 g of m-chloroperbenzoic acid, the mixture was stirred for 10 minutes. The reaction mixture was washed with an aqueous 5% sodium hydrogen solution, dried, and then the solvent was distilled off. The crystals thus formed were recrystallized from ethanol to provide 1.3 g of 6-(3,5-di-tert-butyl-4-hydroxyphenyl)-2,3-dihydroimidazo[2,1-b]thiazole 1-oxide as the 1/2 ethanol addition product.

Melting point: 211-212°C

Mass spectrum: m/e: 346($M^+$)

Elemental analysis for $C_{19}H_{26}N_2O_2S.1/2C_2H_5OH$:

| | C(%) | H(%) | N(%) |
|---|---|---|---|
| Calculated: | 65.11 | 7.78 | 7.82 |
| Found: | 65.01 | 7.91 | 7.58 |

Example 12

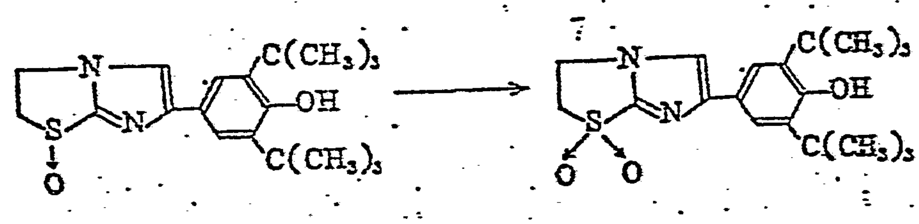

In 10 ml of chloroform was dissolved 0.6 g of 6-(3,5-di-tert-butyl-4-hydroxyphenyl)-2,3-dihydroimidazo[2,1-b]thiazole 1-oxide and then 0.4 g of m-chloroperbenzoic acid was added to the solution. After 30 minutes, 0.4 g of m-chloroperbenzoic acid was further added to the mixture and the resultant mixture was allowed to stand for

one hour. The reaction mixture was washed with an aqueous 5% sodium hydrogen carbonate solution, dried, and then the solvent was distilled off. The residue was purified by silica gel chromatography using chloroform as the eluent and further recrystallized from aqueous ethanol to provide 0.2 g of 6-(3,5-di-tert-butyl-4-hydroxyphenyl)-2,3-dihydroimidazo[2,1-b]-thiazole 1.1-dioxide.

Melting point: 267-269°C

Elemental analysis for $C_{19}H_{26}N_2O_3S$:

| | C(%) | H(%) | N(%) |
|---|---|---|---|
| Calculated: | 62.77 | 7.35 | 7.60 |
| Found: | 62.96 | 7.23 | 7.73 |

Example 13

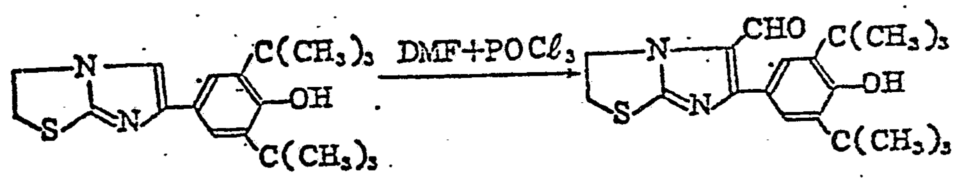


To a solution of 1.5 ml of dimethylformamide in 10 ml of chloroform was added 1.1 g of phosphorus oxychloride under ice-cooling and the mixture was allowed to stand for one hour. To the reaction mixture was added 1.5 g of 6-(3,5-di-tert-butyl-4-hydroxyphenyl)-2,3-dihydroimidazo[2,1-b]thiazole and the mixture was refluxed for 4 hours. To the reaction mixture was added 20 ml of an aqueous 10% potassium carbonate solution and the mixture was stirred for 15 minutes. The chloroform layer was dried and concentrated. The crystals thus obtained were recrystallized

from ethanol to provide 1.0 g of 6-(3,5-di-tert-butyl-4-hydroxyphenyl)-5-formyl-2,3-dihydroimidazo[2,1-b]thiazole.

Melting point: 210-212°C

Elemental analysis for $C_{20}H_{26}N_2O_2S$:

| | C(%) | H(%) | N(%) |
|---|---|---|---|
| Calculated: | 66.82 | 7.20 | 7.80 |
| Found: | 67.01 | 7.31 | 7.81 |

## Example 14

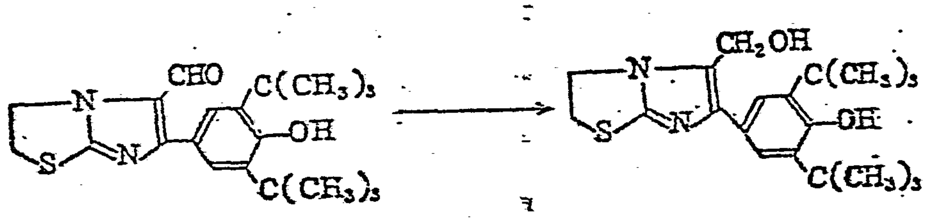

In 10 ml of ethanol was dissolved 0.4 g of 6-(3,5-di-tert-butyl-4-hydroxyphenyl)-5-formyl-2,3-dihydroimidazo[2,1-b]thiazole and after adding 40 mg of sodium borohydride to the solution, the mixture was stirred for 10 minutes. To the reaction mixture were added 0.3 ml of acetic acid and 30 ml of water, and the crystals thus precipitated were recovered by filtration and recrystallized from aqueous ethanol to provide 0.3 g of 6-(3,5-di-tert-butyl-4-hydroxyphenyl)-5-hydroxymethyl-2,3-dihydroimidazo[2,1-b]-thiazole.

Melting point: 227-229°C

Elemental analysis for $C_{20}H_{28}N_2O_2S$:

| | C(%) | H(%) | N(%) |
|---|---|---|---|
| Calculated: | 66.55 | 8.04 | 7.73 |
| Found: | 66.63 | 7.80 | 7.77 |

EXAMPLE 15



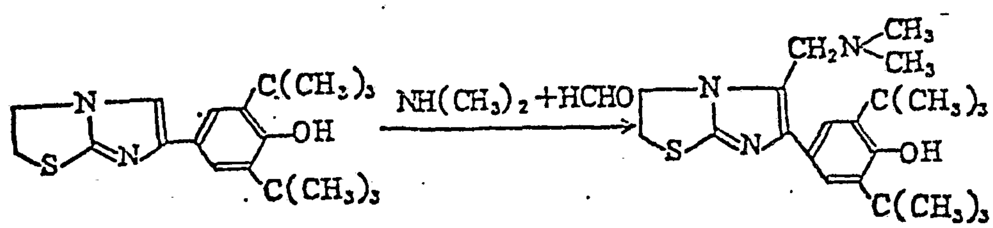


To a mixed solution of 0.9 g of an aqueous 40% dimethylamine solution, 0.6 g of an aqueous 35% formaldehyde solution, 1.5 ml of acetic acid, and 5 ml of dioxane was added 0.66 g of 6-(3,5-di-tert-butyl-4-hydroxyphenyl)-2,3-dihydroimidazo[2,1-b]thiazole and the resultant mixture was refluxed for 6 hours. The reaction mixture was concentrated under reduced pressure and the residue was mixed with 20 ml of an aqueous 10% potassium carbonate solution and extracted with chloroform. The extract was dried and concentrated, and the residue thus formed was purified by silica gel chromatography using chloroform as the eluent and further recrystallized from aqueous ethanol to provide 0.3 g of 5-dimethylaminomethyl-6-(3,5-di-tert-butyl-4-hydroxyphenyl)-2,3-dihydroimidazo[2,1-b]thiazole.1/4$H_2O$.

Melting point: 188-191°C

Mass spectrum: m/e: 387($M^+$)

Elemental analysis for $C_{22}H_{33}N_3OS \cdot 1/4H_2O$:

| | C(%) | H(%) | N(%) |
|---|---|---|---|
| Calculated: | 67.49 | 8.70 | 10.48 |
| Found: | 67.39 | 8.61 | 10.72 |

Example 16

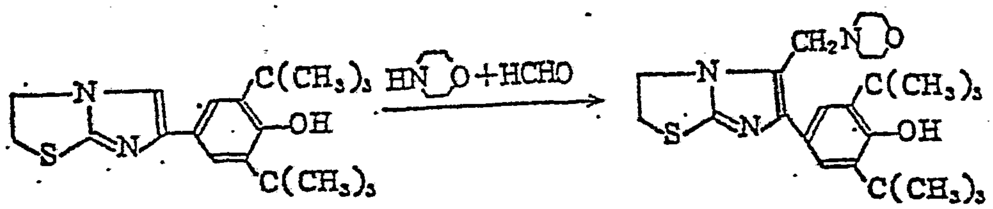

A mixture of 1.65g of 6-(3,5-di-tert-butyl-4-hydroxyphenyl)-2,3-dihydroimidazo[2,1-b]thiazole, 1.8 g of morpholine, 1.7 g of an aqueous 35% formaldehyde solution, 1.5 ml of acetic acid, and 10 ml of dioxane was refluxed for 6 hours. The reaction mixture was concentrated under reduced pressure and to the residue was added 20 ml of an aqueous 10% potassium carbonate to precipitate crystals, which were recovered by filtration and recrystallized from methanol to provide 0.85 g of 6-(3,5-di-tert-butyl-4-hydroxyphenyl)-5-morpholinomethyl-2,3-dihydroimidazo[2,1-b]-thiazole.

Melting point: 231-233°C

Elemental analysis for $C_{24}H_{35}N_3O_2S$:

| | C(%) | H(%) | N(%) | S(%) |
|---|---|---|---|---|
| Calculated: | 67.08 | 8.32 | 9.67 | 7.35 |
| Found: | 67.10 | 8.21 | 9.78 | 7.46 |

Example 17

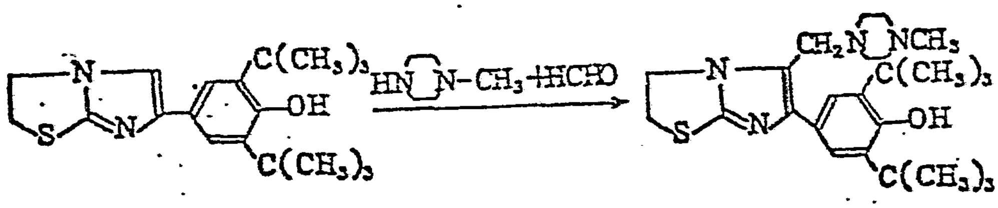

Example 18

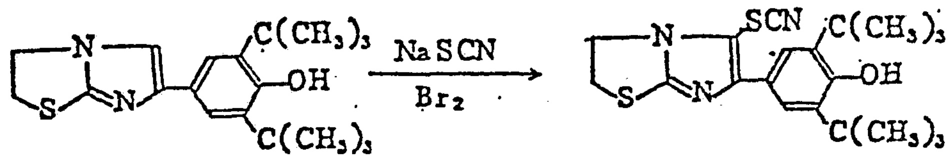


In 10 ml of acetic acid was dissolved 1.35 g of 6-(3,5-di-tert-butyl-4-hydroxyphenyl)-2,3-dihydroimidazo[2,1-b]thiazole and after adding thereto 0.64 g of sodium thiocyanate, 0.7 g of bromine was added dropwise to the solution under ice-cooling. After performing the reaction for one hour at room temperature, 30 ml of water was added to the reaction mixture to precipitate crystals, which were recovered by filtration. The crystals were added to a mixture of 20 ml of chloroform and 10 ml of an aqueous 10% potassium carbonate solution followed by stirring. The chloroform layer thus formed was dried and concentrated, and the residue thus formed was purified by silica gel column chromatography using chloroform as the eluent and further recrystallized

By following the same procedure as in Example 72, 6-(3,5-di-tert-butyl-4-hydroxyphenyl)-5-(4-methylpiperazinomethyl)-2,3-dihydroimidazo[2,1-b]thiazole was produced.

Melting point: 211-212°C

Elemental analysis for $C_{25}H_{38}N_4OS.H_2O$:

| | C(%) | H(%) | N(%) | S(%) |
|---|---|---|---|---|
| Calculated: | 65.28 | 8.61 | 12.00 | 7.06 |
| Found: | 65.18 | 8.75 | 12.16 | 6.96 |

0164765

from ethanol to provide 0.6 g of 6-(3,5-di-tert-butyl-4-hydroxyphenyl)-5-thiocyanato-2,3-dihydroimidazo[2,1-b]thiazole.

Melting point: 178-179°C

Elemental analysis for $C_{20}H_{25}N_3OS_2$:

| | C(%) | H(%) | N(%) | S(%) |
|---|---|---|---|---|
| Calculated: | 62.05 | 6.50 | 10.66 | 16.84 |
| Found: | 61.98 | 6.50 | 10.84 | 16.54 |

Example 19

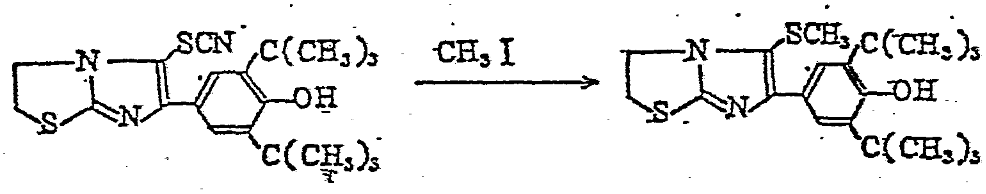


In 10 ml of methanol was dissolved 1.3 g of 6-(3,5-di-tert-butyl-4-hydroxyphenyl)-5-thiocyanato-2,3-dihydroimidazo[2,1-b]-thiazole and after cooling the solution to 0°C, 0.65 g of methyl iodide was added dropwise to the solution with stirring and further a solution of 0.2 g of potassium hydroxide dissolved in a mixture of 2 ml of water and 2 ml of methanol was added dropwise to the solution. After 30 minutes, the reaction mixture was concentrated and the residue was purified by silica gel column chromatography using chloroform as the eluent and further recrystallized from ethanol to provide 0.18 g of 6-(3,5-di-tert-butyl-4-hydroxyphenyl)-5-methylthio-2,3-dihydroimidazo[2,1-b]thiazole.

Melting point: 170-171°C

Elemental analysis for $C_{20}H_{28}N_2OS_2$:

| | C(%) | H(%) | N(%) | S(%) |
|---|---|---|---|---|
| Calculated: | 63.49 | 7.60 | 7.36 | 16.85 |
| Found: | 63.79 | 7.49 | 7.44 | 17.03 |

Example 20

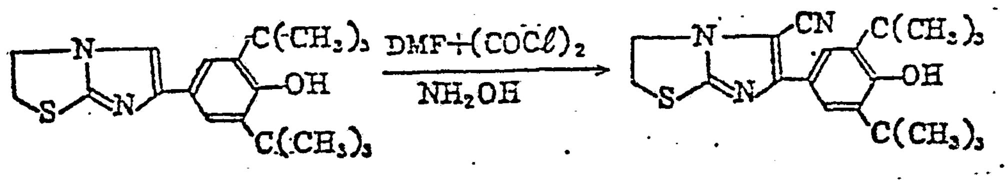


To a mixed solution of 0.5 g of dimethylformamide and 10 ml of ethylene dichloride was added dropwise a solution of 0.9 g of oxalyl chloride in 5 ml of ethylene dichloride under ice-cooling. After allowing the mixture to stand for 15 minutes at room temperature, a solution of 2 g of 6-(3,5-di-tert-butyl-4-hydroxyphenyl)-2,3-dihydroimidazo[2,1-b]thiazole in a mixture of 3 ml of dimethylformamide and 10 ml of ethylene dichloride was added dropwise and the resultant mixture was stirred for 2 hours. Then, a solution of 0.5 g of hydroxylamine hydrochloride dissolved in 1.5 ml of dimethylformamide and 0.6 ml of pyridine was added to the reaction mixture and the resultant mixture was refluxed overnight. The reaction mixture was washed with 30 ml of an aqueous 5% sodium hydrogen carbonate solution, dried, and then concentrated. The residue thus formed was purified by silica gel column chromatography using chloroform as the eluent and further recrytallized

from ethanol to provide 0.33 g of 5-cyano-6-(3,5-di-tert-butyl-4-hydroxyphenyl)-2,3-dihydroimidazo[2,1-b]thiazole.

Melting point: 207-208°C

Elemental analysis for $C_{20}H_{25}N_3OS$:

| | C(%) | H(%) | N(%) |
|---|---|---|---|
| Calculated: | 67.52 | 6.92 | 11.74 |
| Found: | 67.57 | 7.09 | 11.82 |

The clinical doses of the compounds of this invention are usually 10-1,000 mg per day for an adult and the medicament is administered 2 or 3 times per day. The doses are controlled according to the condition and age of the patient.

The compounds of this invention can be administered in various dosage forms such as formulations for oral administration, injections, suppositories for rectal administration, and medicines for topical application.

The compounds of this invention may be used in pharmaceutical compositions prepared by blending with conventional pharmaceutical carriers or excipients in conventional manner. The tablets, capsules, granules, powders, etc., of the compounds of this invention for oral administration may contain conventional pharmaceutical excipients, such as calcium carbonate, calcium phosphate, starch, sucrose, lactose, talc, magnesium stearate,

gelatin, polyvinyl pyrrolidone, gum arabic, sorbitol, microcrystalline cellulose, polyethylene glycol, silica, and sodium laurylsulfate. Moveover, the tablets may be coated as well known in the art. Furthermore, the liquid formulations for oral administration may be aqueous or oily suspensions, syrups, or elixirs, prepared in conventional manner. Suppositories for rectal use may contain a formulation carrier known in the art, such as polyethylene glycol, lanolin, cacao butter or WITEPSOL(TM) (made by Dynamite Nobel Co.)

CLAIMS:

1. A 3,5-di-tert-butyl-4-hydroxyphenyl-substituted heterocyclic compound represented by the following formula I, or a salt thereof :

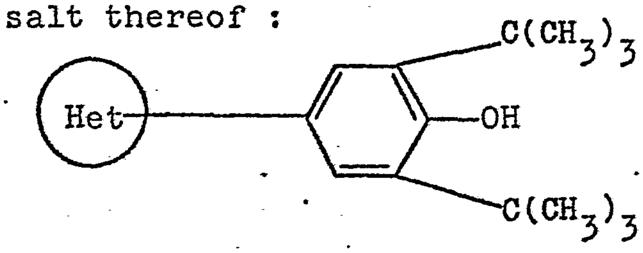

I

wherein Het is a heterocyclic group of the formula

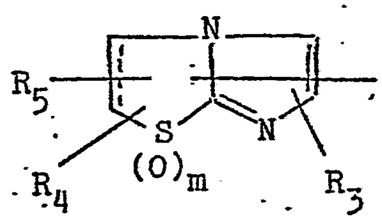

wherein

$R_3$, $R_4$, and $R_5$ are the same or different and selected from hydrogen, halogen, $C_1$ to $C_7$ alkyl groups, hydroxy $C_1$ to $C_7$ alkyl groups, amino $C_1$ to $C_7$ alkyl groups, aryl groups, $C_1$ to $C_7$ alkoxy-substituted aryl groups, $C_1$ to $C_7$ alkanoyl groups, $C_1$ to $C_7$ alkylthio groups, $C_1$ to $C_7$ alkoxy groups, aryl-substituted $C_1$ to $C_7$ alkyl groups, and cyano and thiocyanato groups; m is 0, 1, or 2; and the dotted line means the presence or absence of a double bond.

0164765

2. 6-(3,5-di-tert-butyl-4-hydroxyphenyl)-5-methyl-2,3-dihydroimiazo[2,1-b]thiazole.

3. 6-(3,5-di-tert-butyl-4-hydroxyphenyl)2,3-dihydroimidazo[2,1-b]thiazole.

4. 6-(3,5-di-tert-butyl-4-hydroxyphenyl)-2,3-dihydroimidazo[2,1-b]thiazole 1-oxide or 1,1-dioxide.

5. A pharmaceutical composition containing a compound according to any preceding claim and an excipient.

0164765

6. A process of producing a compound according to claim 1, wherein the compound of formula I is represented by formula $I_b$-1

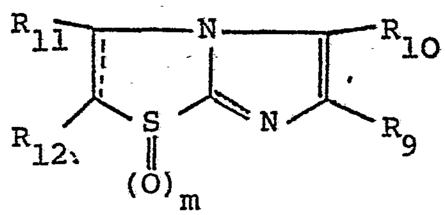

wherein one of $R_9$, $R_{10}$, $R_{11}$ and R is the 3-5-di-tert-butyl-4-hydroxyphenyl group, and the others have the same significance as $R_3$, $R_4$, $R_5$, and m and the dotted line have the same significance as in claim 1, or a salt thereof, which comprises reacting a compound shown by formula $II_b$

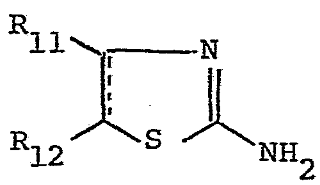

wherein $R_{11}$ and $R_{12}$ are as defined above, with a compound shown by formula $III_b$

$$X - CH - R_{10}$$
$$O = C - R_9$$

wherein X is halogen and $R_9$ and $R_{10}$ are as defined above, and then, if desired, oxidizing, aminomethylating, lower alkanoylating, reducing, cyanating, thiocyanating and/or lower alkylating the product thus obtained.

7. A process of producing a compound according to claim 13 or a salt thereof which comprises reacting a compound shown by formula $IV_b$

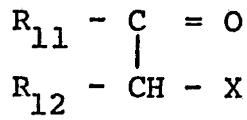

$IV_b$

wherein X is halogen and $R_{11}$ and $R_{12}$ have the same significance as defined in claim 13, with a compound shown by formula $V_b$

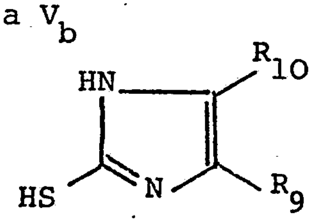

$V_b$

wherein $R_9$ and $R_{10}$ have the same significance as defined in claim 13, and then, if desired, oxidizing, aminomethylating, lower alkanoylating, reducing, cyanating, thiocyanating and/or lower alkylating the resultant product.

European Patent Office

# EUROPEAN SEARCH REPORT

0164765

Application number

EP 85200531.3

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl 4) |
| D,A | EP - A2/A3 - 0 000 353 (CIBA-GEIGY AG)<br>* Abstract *<br>-- | 1,5 | C 07 D 513/04<br>A 61 K 31/425<br>A 61 K 31/415 |
| A | US - A - 4 104 400 (WEISENBORN et al.)<br>* Abstract *<br>-- | 1 | |
| A | AU - A - 475 623 (AMERICAN CYANAMID COMPANY)<br>* Claim 1 *<br>---- | 1 | |
| | | | TECHNICAL FIELDS SEARCHED (Int. Cl.4) |
| | | | C 07 D 513/00 |

The present search report has been drawn up for all claims

| Place of search | Date of completion of the search | Examiner |
|---|---|---|
| VIENNA | 08-08-1985 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document